# EUROPÄISCHE PATENTSCHRIFT

(11) **EP 3 415 193 B1**
(45) Veröffentlichungstag und Bekanntmachung des Hinweises auf die Patenterteilung: **10.12.2025**
(21) Anmeldenummer: 17175471.6
(22) Anmeldetag: 12.06.2017
(51) Int. Cl.: A61M 37/00

(54) **NADELFÜHRUNGSVORRICHTUNG FÜR EINE HAUTSTECHEINRICHTUNG UND HAUTSTECHEINRICHTUNG**
NEEDLE GUIDE FOR A SKIN PUNCTURING DEVICE AND SKIN PUNCTURING DEVICE
DISPOSITIF D'INTRODUCTION D'AIGUILLE POUR UN DISPOSITIF DE PIQUAGE DE PEAU ET DISPOSITIF DE PIQUAGE DE PEAU

(43) Veröffentlichungstag der Anmeldung: 19.12.2018
(73) Patentinhaber: MT.DERM GmbH, 14167 Berlin (DE)
(72) Erfinder: Veil, Tobias, 12103 Berlin (DE); Holland, Andy, 12109 Berlin (DE)
(74) Vertreter: Bittner, Thomas L.

(56) Entgegenhaltungen:
- CN-Y- 2 517 389
- DE-A1- 102014 011 438
- JP-A- 2008 125 830
- US-A1- 2005 066 775
- US-A1- 2014 358 172
- US-A1- 2019 029 120

## Beschreibung

Die Erfindung betrifft eine Nadelführungsvorrichtung für eine Hautstecheinrichtung und eine Hautstecheinrichtung zum Aufstechen einer menschlichen oder tierischen Haut.

### Hintergrund

Hautstecheinrichtungen, also Vorrichtungen zum lokalen Aufstechen einer menschlichen oder einer tierischen Haut, werden in der Regel als Handgeräte ausgeführt. Vom Bedienpersonal können derartige Handgeräte zum Aufbringen einer Farbe für eine Tätowierung (Tattoo) und / oder permanentes Make-up im Bereich der Hautoberfläche verwendet werden. Aber auch das Einbringen kosmetischer oder medizinischer Wirkstoffe über die Haut ist mit solchen Geräten möglich, indem die Haut lokal aufgestochen wird. Darüber hinaus können solche Geräte verwendet werden, ohne dass irgendeine Substanz eingebracht wird, zum Beispiel zur Hautstimulierung.

Ein Handgerät zum lokalen Aufstechen einer Haut ist beispielsweise aus der Druckschrift DE 299 19 199 U1 bekannt. Das bekannte Handgerät weist ein Griffstück, eine Antriebseinrichtung und eine Stechnadel auf, die mit Hilfe der Antriebseinrichtung im Betrieb relativ zu einer Nadeldüse vor und zurück bewegt wird, wobei mindestens zwei lösbar miteinander verbundene Module vorgesehen sind, und das eine der beiden Module als wieder verwendbares Grundmodul mit integrierter Antriebseinrichtung ausgebildet ist. Das andere der beiden Module ist ein sterilisiertes Einwegmodul, in das bei dem bekannten Handgerät alle durch die Körperflüssigkeiten eines Kunden infizierbaren Bestandteile integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches die Antriebseinrichtung umfasst, wieder verwendet wird. Mit Hilfe des Einwegmoduls werden die hygienischen Bedingungen beim Aufbringen einer Tätowierung und / oder von permanentem Make-up verbessert, da alle Teile ausgetauscht werden, die potentiell durch die bei der Behandlung austretende Körperflüssigkeit des Kunden kontaminiert werden können. Es wird so vermieden, dass das gesamte Handgerät ausgetauscht werden muss.

Aus dem Dokument EP 1 495 782 A1 ist ein Antriebsmodul für eine Vorrichtung zum lokalen Aufstechen einer menschlichen oder einer tierischen Haut bekannt, bei dem eine Antriebseinrichtung, mit der eine Antriebsbewegung erzeugbar ist, und ein an die Antriebseinrichtung gekoppelter Wandlungsmechanismus vorgesehen sind, mit dem die Antriebsdrehbewegung in eine auf eine die Haut lokal aufstechende Stecheinrichtung einkoppelbare Vorwärts- / Rückwärtsbewegung umgewandelt wird, so dass eine repetierende Bewegung einer Haustechnadel ermöglicht ist. Der Wandlungsmechanismus umfasst ein Funktionsbauteil, welches bei der Bewegungswandlung eine Taumel- oder Kippbewegung ausführt, wodurch eine Antriebskraft zum Bewegen einer die Haut lokal aufstechenden Nadel in Vorwärts- und Rückwärtsrichtung zur Verfügung gestellt wird.

Das Dokument US 2014/0358172 A1 betrifft eine Nadelmontagestruktur, die einen Griffabschnitt mit einem Aufnahmeraum zum Aufnehmen eines Nadelantriebsmechanismus und einer Öffnung an einem unteren Ende davon in Verbindung mit dem Aufnahmeraum aufweist, sodass eine Tätowierungsnadel des Nadelantriebsmechanismus außerhalb der Öffnung freigelegt werden kann. Des Weiteren ist ein Tätowierungsnadelführungsrohr vorgesehen, das zwei Enden jeweils mit Durchgangsloch aufweist. Die zwei Durchgangslöcher stehen miteinander in Verbindung, und die Innenwand des Tätowierungsnadelführungsrohrs ist axial mit einer Vielzahl von Nuten ausgebildet, die jeweils zwei Enden haben, welche sich jeweils zu den beiden Durchgangslöchern erstrecken.

In dem Dokument US 2019/029120 A1 sind Nadelstifte und Verfahren zum Markieren und/oder Erstellen von Artikeln und Gegenständen sowie für weitere Verwendungen beschrieben.

Das Dokument DE 10 2014 011 438 A1 offenbart eine Tätowierungsnadel mit einem sich entlang einer in Längsrichtung verlaufenden Längsachse erstreckenden Nadelkörper, der wenigstens an einem Ende in Längsrichtung in einer Verjüngung ausläuft, die dazu ausgebildet ist, die Tätowierungsnadel wenigstens teilweise in die Haut einzuführen. Zur verbesserten Farbabgabe ist wenigstens ein Freischliff vorgesehen, um einen Teil von Farbmittel darin aufnehmen, insbesondere bevorraten zu können. Der Freischliff ist als Ausnehmung in die Oberfläche eingebracht und erstreckt sich zumindest teilweise senkrecht zur Längsachse.

Das Dokument JP 2008 125 830 A betrifft eine Tätowierungsnadel, die mit einer Flüssigkeitsströmungsführungsnut auf der Oberfläche eines geneigten Abschnitts der Tätowierungsnadel ausgebildet ist. Vorzugsweise ist die Flüssigkeitsströmungsführungsnut parallel zur Achse der Tätowierungsnadel angeordnet. Die Flüssigkeitsströmungsführungsnut ist eine spiralförmige Nut, die zur Achse der Tätowierungsnadel geneigt ist.

Das Dokument CN 2 517 389 Y bezieht sich auf eine verbesserte Nadelbuchse einer Augenbrauen-Tätowierungsmaschine. Am Boden der Nadelbuchse ist ein vertiefter, trichterförmiger Pigmentführungsauslass angeordnet, um das Pigment während des Augenbrauen-Tätowierens ohne Auslaufen zu halten und ein geeignetes Pigment für das Durchstechen in die Haut leicht herauszuführen. In der Nadelbuchse sind eine Augenbrauen-Tätowierungsnadelführungsnut und eine Pigmentführungskapillarnut angeordnet.

Das Dokument US 2005/066775 A1 offenbart eine Nadel zum Tätowieren der Haut oder der Augenbraue. Die Nadel umfasst ein scharfes Ende mit einem sich verjüngenden Abschnitt mit einer Vielzahl von erhabenen Elementen. Durch Punktieren der Haut oder der Augenbraue mit der Nadel bildet sich ein kreisförmiges Loch mit einer Kante mit einer Vielzahl von Einkerbungen darum, damit flüssiger Farbstoff vollständig aus dem sich verjüngenden Abschnitt darin strömen kann.

Bei bekannten Vorrichtungen ist die Hautstechnadel in einem Nadelmodul aufgenommen, welches an das Antriebsmodul koppelt, um die bereitgestellte Antriebsbewegung oder -kraft zum Vor- und Zurückbewegen auf die Hautstechnadel zu koppeln, welche bei dieser Bewegung in dem Nadelmodul geführt wird.

### Zusammenfassung

Aufgabe der Erfindung ist es, eine Nadelführungsvorrichtung für eine Hautstecheinrichtung und eine Hautstecheinrichtung anzugeben, mit denen eine verbesserte Führung für die Stechnadel bereitgestellt ist.

Zur Lösung der Aufgabe sind eine Nadelführungsvorrichtung für eine Hautstecheinrichtung sowie eine Hautstecheinrichtung nach den unabhängigen Ansprüchen 1 und 12 geschaffen. Alternative Ausgestaltungen sind Gegenstand von abhängigen Unteransprüchen.

Nach einem Aspekt ist eine Nadelführungsvorrichtung für eine Hautstecheinrichtung geschaffen, die einen Basiskörper und eine Stech- oder Hautstechnadel sowie einen Nadelkanal aufweist, welcher in dem Basiskörper gebildet und in dem die Hautstechnadel angeordnet ist. Die Hautstechnadel ist in dem Nadelkanal entlang einer Bewegungsbahn repetierend hin und her bewegbar. Eine Kanalwand des Nadelkanals weist entlang der Bewegungsbahn in Bezug auf die Hautstechnadel proximale Führungswandabschnitte, an denen die Hautstechnadel beim Hin- und Herbewegen zur Nadelführung zur Anlage kommt, und distale Wandabschnitte auf, die gegenüber dem proximalen Führungswandabschnitten zurückversetzt und beim Hin- und Herbewegen der Hautstechnadel im Nadelkanal frei von einem Berührungskontakt mit der Hautstechnadel sind.

Nach einem weiteren Aspekt ist eine Hautstecheinrichtung mit einem Antriebsmodul und einem hieran gekoppelten Nadelmodul geschaffen, wobei das Nadelmodul eine Nadelführungsvorrichtung der vorgenannten Art aufweist.

Der Basiskörper kann ein- oder mehrteilig ausgeführt sein. Zum Beispiel kann ein ein- oder mehrteiliges Gehäuse vorgesehen sein, beispielweise eine Gehäuse mit Gehäusedeckel.

Die Hautstechnadel kann als eine Einzelnadel oder als eine Nadelgruppe ausgeführt sein, in der mehrere Einzelnadeln zur Hautstechnadel zusammengefasst sind. Die Einzelnadel(n) können im Querschnitt ein Rund- oder ein Flachprofil aufweisen.

Hautstecheinrichtungen, insbesondere in Form von Handgeräten, mit einem Antriebsmodul und einem hieran gekoppelten Nadelmodul sind als solche in verschiedenen Ausführungsformen bekannt. Beispielsweise kann das Antriebsmodul eine repetierende Antriebsbewegung zur Verfügung stellen, wobei als Antrieb ein Elektromotor nutzbar ist. Die Antriebsbewegung wird über einen Kopplungsmechanismus auf einen Nadelschaft in dem Nadelmodul gegeben, um die am Nadelschaft aufgenommene Hautstechnadel vor und zurück zu bewegen, wobei eine Nadelspitze der Hautstechnadel zumindest in einer ausgefahrenen Stellung außerhalb einer vorderseitigen Öffnung des Nadelmoduls angeordnet ist, die auch als Nadelöffnung bezeichnet wird. Die Nadelöffnung kann an einer Nadeldüse gebildet sein, die am Nadelmodul vorderseitig angeordnet ist, beispielweise als lösbare und somit austauschbare Nadeldüse. Es kann vorgesehen sein, dass die Antriebseinrichtung eingerichtet ist, im Betrieb eine Antriebsbewegung mit einer Wiederholfrequenz zwischen etwa 30 Hz und etwa 160 Hz bereitzustellen.

Die Hautstecheinrichtung kann als ein Handgerät ausgebildet sein, bei dem am Gehäuse ein Handgriff ausgebildet ist.

Das Nadelmodul mit der Nadelführungsvorrichtung kann als ein Einwegmodul ausgebildet sein, welches lösbar an dem Antriebsmodul angebracht ist.

Bei der Ausführung mit wechselbarer Modul- oder Nadeldüse kann vorgesehen sein, dass mit ein und demselben Nadelmodul gleichartige Modul- oder Nadeldüsen oder verschiedenartige Modul- oder Nadeldüsen zum Einsatz kommen. Eine Modul- oder Nadeldüse kann aus unterschiedlichen Materialien bestehen, beispielsweise Kunststoff und / oder Metall. Eine Ausführung als Spritzgussbauteil kann vorgesehen sein. Die Modul- oder Nadeldüse kann ein- oder mehrteilig ausgebildet sein, zum Beispiel mithilfe von mehreren zusammensteckbaren Düsenbauteilen.

Die Nadelführungsvorrichtung kann vollständig oder teilweise in der Modul- oder Nadeldüse ausgebildet sein.

Die proximalen Führungswandabschnitte und die distalen Wandabschnitte sind entlang der Bewegungsbahn in dem Nadelkanal abwechselnd gebildet. Hierbei kann auf einen proximalen Führungswandabschnitt stets ein distaler Wandabschnitt folgen und umgekehrt. Alternativ können auch mehrere proximale Führungswandabschnitte zwischen mehreren distalen Wandabschnitten gebildet sein.

Die proximalen Führungswandabschnitte und / oder die distalen Wandabschnitte sind entlang einer oder mehrerer jeweils um den Nadelkanal verlaufender Spirallinien angeordnet. Die Spirallinie kann als Schraubenlinie ausgeführt sein, also eine Spirallinie mit gleichbleibender Steigung. Hierbei können mehrere Spiral- oder Schraubenlinien vorgesehen sein, die jeweils einem proximalen Führungswandabschnitt oder einem distalen Wandabschnitt zugeordnet sind. Bei dieser Ausgestaltung erstreckt sich die Strukturierung der Kanalwand mit den proximalen Führungswandabschnitten und den distalen Wandabschnitten vollständig um die Hautstechnadel herum.

In einer anderen Ausgestaltung können ergänzend die proximalen Führungswandabschnitte und / oder die distalen Wandabschnitte entlang einer oder mehrerer mäanderartig geschwungener Linien entlang des Nadelkanals angeordnet sein.

Der Nadelkanal kann als ein Fluidtransportkanal ausgeführt sein. Bei dieser Ausgestaltung ist der Nadelkanal ergänzend als Fluidtransportkanal eingerichtet, um beim Hin- und Herbewegen der Hautstechnadel ein in die Haut einzubringendes Fluid, beispielsweise eine Farbe, von einem Kanaleintritt des Nadelkanals zu einem Kanalaustritt des Nadelkanals zu transportieren. Dies kann beispielsweise dadurch erfolgen, dass das Fluid beim Hin- und Herbewegen der Hautstechnadel Stück für Stück zum Kanalaustritt hin transportiert wird, indem die Nadelbewegung das Fluid zwischen benachbarten Volumina überträgt, die im Bereich der distalen Wandabschnitte diesen vorgelagert sind. Auf diese Weise wird das Fluid entlang der Bewegungsbahn Stück für Stück vorwärts transportiert, um schließlich zum Kanalaustritt zu gelangen.

Den distalen Wandabschnitten vorgelagerte Volumina können entlang der Bewegungsbahn in Fluidverbindung stehen. Bei dieser Ausführungsform erfolgt der Transport des Fluids entlang des Fluidtransportkanals, indem das Fluid zwischen den benachbarten Volumina fließt. Ergänzend kann der durch die Bewegung der Hautstechnadel verursachte Transport wirken. Es ist eine durchgehende Fluidverbindung zwischen dem Kanaleintritt und dem Kanalaustritt ausgebildet. Insbesondere wird das Fluid entlang distaler Wandabschnitte transportiert, die sich entlang einer die Bewegungsbahn umgreifenden Spirale erstrecken.

Die Bewegungsbahn kann einen geraden Abschnitt in einem geraden Nadelkanalabschnitt und / oder einen gekrümmten Abschnitt in einem gekrümmten Nadelkanalabschnitt aufweisen. Die Bewegungsbahn in dem Nadelkanal kann vollständig gerade oder vollständig gekrümmt ausgebildet sein. Die jeweilige Bewegungsbahn kann eingerichtet sein, die Hautstechnadel beim Austreten aus einer Nadeldüsenöffnung parallel oder schräg zur Längsrichtung des Basiskörpers mit dem Nadelkanal zu bewegen.

Die proximalen Führungswandabschnitte können eine flächige und / oder eine punktförmige Kontaktfläche aufweisen, derart, dass beim Führen der Hautstechnadel in dem Nadelkanal diese mit dem proximalen Führungswandabschnitt einen flächigen und / oder einen punktförmigen Berührungskontakt ausbildet. Die flächige Kontaktfläche kann entlang einer linienförmigen Kontaktfläche verlaufen. Die unterschiedlichen Kontaktflächen können an proximalen Führungswandabschnitten gebildet sein, die einen dreieckigen oder viereckigen Querschnitt aufweisen. Auch eine der Hautstechnadel zugewandte gerundete Oberfläche im Bereich des proximalen Führungswandabschnitts kann vorgesehen sein. Die in dem Nadelkanal ausgebildeten Kontaktflächen können alle gleich oder verschieden ausgeführt sein. Hierbei können sich die Kontaktflächen von zueinander benachbart und / oder einander gegenüberliegenden angeordneten proximalen Führungswandabschnitten unterscheiden. In den verschiedenen Ausführungsformen können die proximalen Führungswandabschnitte und die distalen Wandabschnitte entlang einer wellenförmigen Kanalwand ausgebildet sein, wobei die Wellenberge und Wellentäler vergleichbar einer Sinuswelle oder eine Dreieckswelle ausgebildet sein können.

Die proximalen Führungswandabschnitte und / oder die distalen Wandabschnitte in einander gegenüberliegenden Kanalwandabschnitten entlang der Bewegungsbahn können versetzt zueinander ausgebildet sein. In einer Ausführungsform kann ein schlangenförmiger Nadelkanal ausgebildet sein.

Der Nadelkanal kann mit einem Fluidreservoir verbunden sein. Die Verbindung zum Fluidreservoir kann rückseitig einer Nadeldüse an den Nadelkanal koppeln. Das Fluidreservoir kann in dem Nadelmodul selbst angeordnet sein.

Eine effektive Kanalbreite des Nadelkanals, die durch einen Abstand quer zur Längsrichtung des Nadelkanals von proximalen Führungswandabschnitten auf gegenüberliegenden Seiten des Nadelkanals bestimmt ist, kann größer als eine Dicke der Hautstechnadel sein, insbesondere derart, dass eine leichtgängiges Bewegen der Hautstechnadel in dem Nadelkanal gegeben ist, insbesondere auch dann, wenn entlang des Nadelkanals das Fluid transportiert wird. Die effektive Kanalbreite kann über die Länge des Nadelkanals gleichbleibend sein. Alternativ kann sich die effektive Kanalbreite auf der Länge des Nadelkanals ändern. Die Nadeldüse kann an einem Nadelmodul vorgesehen sein, welches lösbar an ein Antriebsmodul eines Handgeräts zum lokalen Aufstechen einer menschlichen oder tierischen Haut gekoppelt ist. Der Kanalaustritt kann die Austrittsöffnung an einer Spitze des Nadelmoduls bilden.

Die effektive Nadelkanalbreite (effektiver Nadelkanaldurchmesser) kann kleiner als die Breite (der Durchmesser) eines minimalen Austragungsvolumens sein, welches durch einen minimalen Abstand quer zur Längsrichtung des Nadelkanals von proximalen Führungswandabschnitten und gegenüberliegenden distalen Wandabschnitten bestimmt ist. Hierdurch kann die Möglichkeit geschaffen sein, über die Einstellung der Breite (des Durchmessers) des Austragungsvolumens den Fluss des auszutragenden Fluids, beispielweise eine Farbe, zu regulieren und gleichzeitig eine präzise Nadelführung durch eine enge effektiven Nadelkanalbreite zu bieten. Es kann so eine präzise / enge Führung der Hautstechnadel bei gleichzeitig "hohem" Fluidfluss erreicht werden.

Der Nadelkanal kann zumindest teilweise in einer Nadeldüse ausgebildet sein.

Der Nadelkanal kann zumindest teilweise in einem Nadelführungsbauteil eines Nadelmoduls für eine Haustecheinrichtung ausgebildet sein. Das Nadelführungsbauteil kann rückwertig von der Modulspitze mit der Nadelöffnung des Nadelmoduls im Gehäuse des Nadelmoduls angeordnet sein, beispielsweise derart, dass zwischen dem Kanalaustritt an dem Nadelführungsbauteil und einer Führung der Hautstechnadel im Bereich der Modulspitze ein freiliegender Abschnitt der Hautstechnadel gebildet ist, welcher nicht in einem Nadelführungskanal angeordnet ist. Das Nadelführungsbauteil kann als lösbares Bauteil im Gehäuse des Nadelmoduls angeordnet sein.

Der Basiskörper kann mit dem Nadelkanal als ein Einwegbauteil ausgeführt sein. Eine solche Ausführung kann beispielsweise im Zusammenhang mit einer auswechselbaren Nadel- oder Moduldüse vorgesehen sein.

In einer alternativen Ausführungsform der Nadeldüse kann im Bereich der Nadelöffnung ein Nadelführelement angeordnet sein, welches beispielhaft als Vorsatz oder Aufsatz ausgeführt ist und in welchem die Hautstechnadel beim Vor- und Zurückbewegen in einer Verlängerung des Nadelkanals geführt ist. Das Nadelführungselement kann von der Nadeldüse abstehen und mit einem proximalen Ende in die Nadelöffnung eingesetzt oder eingesteckt sein. Alternative kann das proximale Ende auf das Nadelführelement aufgesetzt oder einstückig an der Nadeldüse angeformt sein. Eine Austrittsöffnung für die Hautstechnadel kann so versetzt zur Nadelöffnung angeordnet sein. Das Nadelführelement kann frei von proximalen Führungswandabschnitte und distalen Wandabschnitte gebildet sein.

In einer weiteren alternativen Ausführungsform der Nadeldüse kann im Bereich der Nadelöffnung ein Nadelführelement angeordnet sein, welches als Ansatz, Einsatz oder Aufsatz ausgeführt ist und in welchem die Hautstechnadel beim Vor- und Zurückbewegen in dem Nadelkanal geführt ist. Das Nadelführelement kann von der Nadeldüse nach vorn ab- oder überstehen und kann mit einem proximalen Ende in die Nadelöffnung eingesetzt sein. Alternativ kann das Nadelführelement aufgesetzt sein. Auch kann eine einstückige Ausführung mit der Nadeldüse vorgesehen sein. An dem Nadelführelement können im Bereich der Kanalwand die proximalen Führungswandabschnitte und die distalen Wandabschnitte entlang des Nadelkanals gebildet sein. Eine Austrittsöffnung für die Hautstechnadel kann versetzt (vorgelagert) zur Nadelöffnung angeordnet sein. Im Abschnitt mit der Nadelöffnung kann rückseitig ein Führungsabschnitt angeordnet sein, welcher an das Nadelführungselement anschließt. Alternativ kann ein Abstand hierzwischen vorgesehen sein.

### Beschreibung von Ausführungsbeispielen

Im Folgenden werden Ausführungsbeispiele unter Bezugnahme auf Figuren einer Zeichnung erläutert. Hierbei zeigen:
- Fig. 1: eine schematische Darstellung einer als Handgeräts zum lokalen Aufstechen einer menschlichen oder einer tierischen Haut ausgeführten Hautstecheinrichtung, beispielsweise zum Herstellen eines Tattoos oder von permanentem Make-up;
- Fig. 2: eine schematische Darstellung eines Nadelmoduls, bei dem ein Nadelführungsbauteil im Gehäuse des Nadelmoduls angeordnet ist;
- Fig. 3: eine schematische Darstellung eines Nadelmoduls, bei dem das Nadelführungsbauteil näher zur Modulspitze angeordnet ist;
- Fig. 4: einen vorderen Abschnitt eines Nadelmoduls mit einem Nadelkanal in einer Nadeldüse, wobei eine Hautstechnadel eingefahren ist;
- Fig. 5: eine weitere schematische Darstellung der Anordnung aus Fig. 4, wobei die Nadelspitze der Hautstechnadel ausgefahren ist;
- Fig. 6: eine schematische Darstellung einer Nadeldüse mit einem Nadelkanal;
- Fig. 7: eine schematische Darstellung einer weiteren Nadeldüse mit Nadelkanal;
- Fig. 8: eine schematische Darstellung einer anderen Nadeldüse mit Nadelkanal;
- Fig. 9: eine schematische Darstellung einer weiteren Nadeldüse mit Nadelkanal;
- Fig. 10: eine schematische Darstellung eines Nadelführungsbauteils mit Nadelkanal;
- Fig. 11: eine schematische Darstellung eines weiteren Nadelführungsbauteils mit Nadelkanal;
- Fig. 12: eine schematische Darstellung eines vorderen Abschnitts eines Nadelmoduls für eine Flach- oder eine Rundnadel;
- Fig. 13: eine schematische Darstellung eines vorderen Abschnitts eines weiteren Nadelmoduls für eine Flach- oder eine Rundnadel;
- Fig. 14: eine schematische Darstellung von Nadelkanälen im Querschnitt;
- Fig. 15: schematische Darstellungen von Abschnitten eines Nadelkanals;
- Fig. 16: schematische Darstellungen für weitere Ausführungsformen eines Nadelkanals;
- Fig. 17: eine schematische perspektivische Darstellung einer Nadeldüse mit einem Nadelkanal, bei der Führungsabschnitte entlang einer Spirallinie angeordnet sind;
- Fig. 18: eine schematische perspektivische Darstellung einer weiteren Nadeldüse mit einem Nadelkanal, bei der Führungsabschnitte entlang einer mäanderartigen Linie angeordnet sind;
- Fig. 19: eine schematische Darstellung einer alternativen Ausführungsform einer Nadeldüse und
- Fig. 20: eine schematische Darstellung einer weiteren alternativen Ausführungsform einer Nadeldüse.

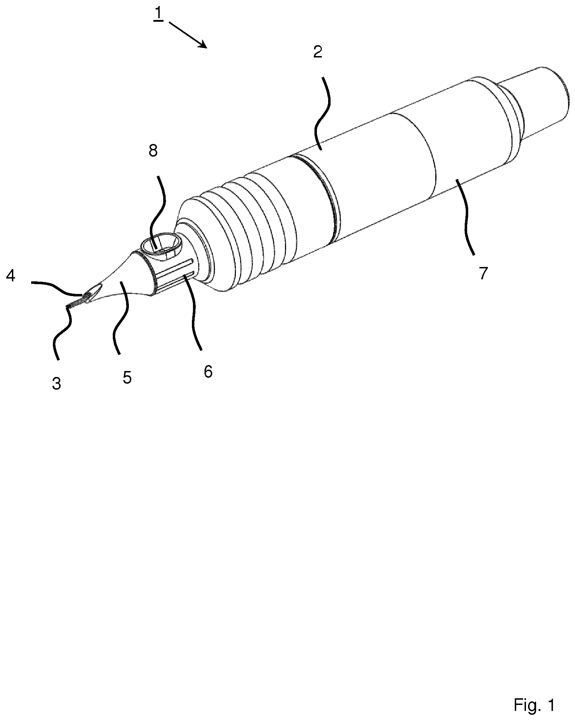

Fig. 1 zeigt eine perspektivische Darstellung einer als Handgerät ausgeführten Hautstecheinrichtung 1, welche modulartig aufgebaut ist. In einem Gehäuse 2, welches hier modulartig oder mehrteilig ausgeführt ist, sind eine Antriebseinrichtung sowie ein Kopplungsmechanismus (nicht dargestellt) aufgenommen, mit dem eine von der Antriebseinrichtung, beispielsweise einem Elektromotor, erzeugte Antriebskraft auf eine Stecheinrichtung gekoppelt wird, die bei der dargestellten Ausführungsform mit einer Stech- oder Hautstechnadel 3 ausgeführt ist, die eine Nadelgruppe aufweist. Die Hautstechnadel 3 ist in Fig. 1 in einer ausgefahrenen Stellung gezeigt. In der ausgefahrenen Stellung steht die Hautstechnadel 3 über eine Nadelöffnung 4 an einer Nadeldüse 5 eines Nadelmoduls 6 hinaus. Das Nadelmodul 6 koppelt an ein Antriebsmodul 7, welches die Antriebseinrichtung in dem Gehäuse 2 aufweist. Die Hautstecheinrichtung 1 kann mit einem Steuergerät (nicht dargestellt) kombiniert werden, um die Antriebseinrichtung im Betrieb mit Energie zu versorgen und die Stechbewegungen zu steuern, also das Aus- und Einfahren der Stechnadel 3.

Ein einzubringendes Fluid, beispielweise eine Farbe oder ein medizinischer oder kosmetischer Stoff, können über eine Öffnung 8 eingebracht werden, um das Fluid dann über die Nadel- oder Nadeldüsenöffnung 4 zur Haut hin auszubringen. Alternativ oder ergänzend kann im und / oder am Gehäuse 2 ein Fluidreservoir (nicht dargestellt) vorgesehen sein, welches mit der Nadelöffnung 4 in Fluidverbindung steht.

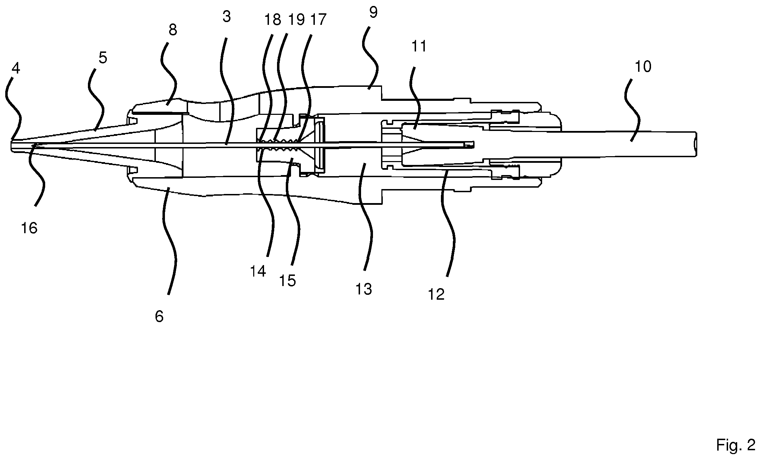

Fig. 2 zeigt ein Nadelmodul 6 mit einem Nadelmodulgehäuse 9, in dem die Hautstechnadel 3 angeordnet ist, die rückseitig an einem Nadelschaft 10 aufgenommen ist. Der Nadelschaft 10 ist mit einem vorderen Abschnitt 11 in einer elastischen Membran 12 aufgenommen. Die elastische Membran 12 dichtet einen Innenraum 13 im Nadelmodulgehäuse 9 gegenüber dem Nadelschaft 10 und den diesen aufnehmenden Raum ab.

Die Hautstechnadel 3 ist entlang eines Nadelkanals 14 in einem Nadelführungsbauteil 15 geführt und tritt beim Vor- und Zurückbewegen zumindest mit einer Nadelspitze 16 durch die Nadeldüsenöffnung 4 an der Nadel- oder Moduldüse 5 aus.

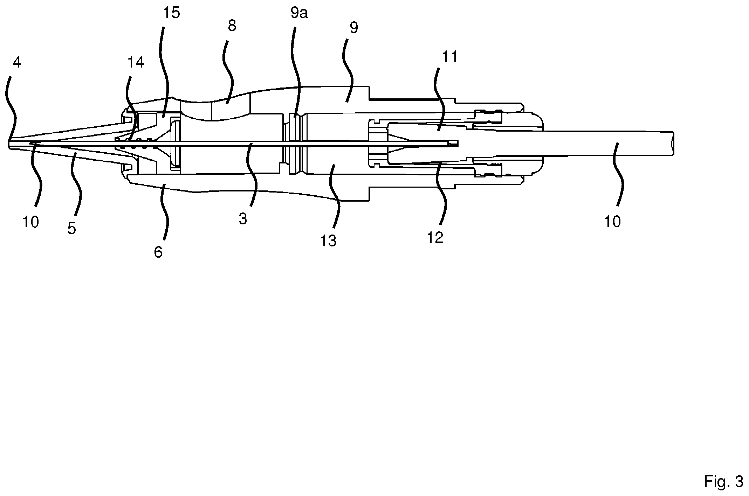

Fig. 3 zeigt eine schematische Darstellung eines Nadelmoduls 6, bei dem das Nadelführungsbauteil 15 auf einer Rückseite der Nadeldüse 5 benachbart hierzu angeordnet ist und teilweise in die Nadeldüse 5 eingreift.

In dem Nadelführungsbauteil 15 ist der Nadelkanal 14 gebildet, der an einer Kanalwand 17 proximale Führungswandabschnitte 18 und distale Wandabschnitte 19 aufweist. Beim Vor- und Zurückbewegen der Hautstechnadel wird diese mittels der proximalen Führungswandabschnitte 18 geführt, derart, dass die proximalen Führungswandabschnitte potentiell mit der Hautstechnadel in Berührungskontakt kommen, sodass die Hautstechnadel hier durchgeführt wird. Zwischen den proximalen Führungswandabschnitten 18 sind demgegenüber zurückversetzte distale Wandabschnitte 19 vorgesehen. An diesen kommt die Hautstechnadel in der Bewegung nicht zur Anlage. Vielmehr sind zwischen der Hautstechnadel und den distalen Wandabschnitten 19 Volumina 20 ausgebildet, die potentiell zur Aufnahme eines Fluids dienen können, welches durch die Nadelöffnung 4 ausgebracht wird.

In dem Nadelmodul 6 ist die Hautstechnadel 3 zusätzlich an einem Gehäuseabschnitt 9a geführt.

Das Nadelführungsbauteil 15 ist bei der Ausführung in Fig. 2 in Bezug auf die Öffnung 8 rückwärtig angeordnet, während es bei der Ausführung in Fig. 3 zwischen Nadelöffnung 4 und Öffnung 8 angeordnet ist. Wenn über die Öffnung 8 ein auf die Haut zu applizierendes Fluid eingebracht wird, wird dieses bei der Ausführung in Fig. 3 durch den Fluidkanal 14 transportiert, insbesondere aufgrund der Hin- und Herbewegung der Hautstechnadel 3, um schließlich zu der Nadelöffnung 4 zu gelangen. Der Nadelkanal 14 ist dann insbesondere bei dieser Ausgestaltung als ein Fluidtransportkanal ausgebildet.

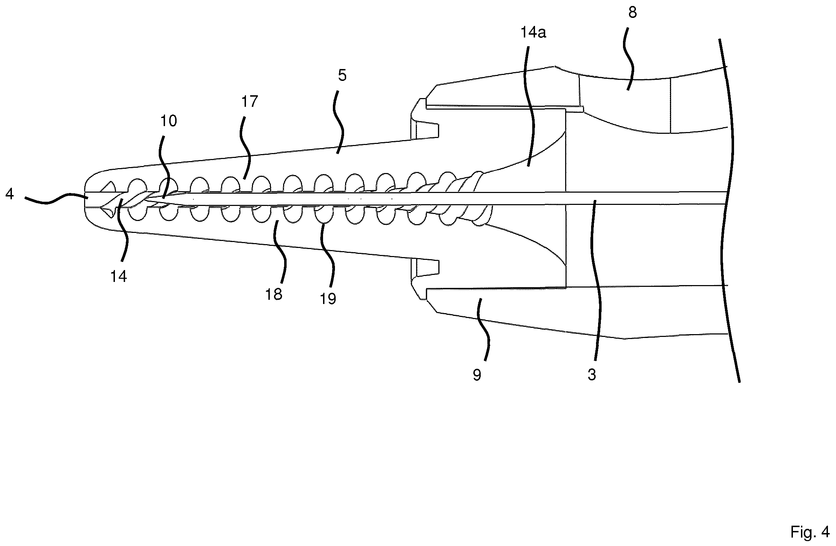

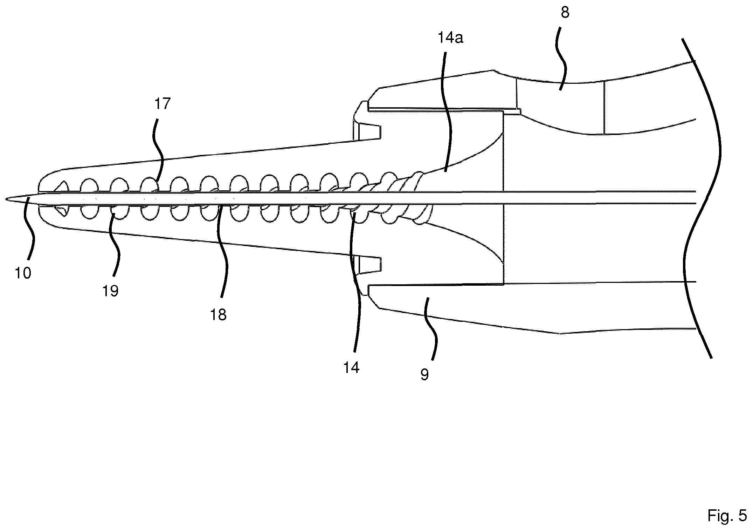

Die Fig. 4 und 5 zeigen einen vorderen Abschnitt des Nadelmoduls 6, wobei der Nadelkanal 14 in der Nadeldüse 5 gebildet ist, welche hier das Nadelführungsbauteil 15 bereitstellend ausgeführt ist. In einem rückwärtigen Abschnitt 14a ist eine Aufweitung angeordnet, die rückseitig an den Nadelkanal 14 anschließt.

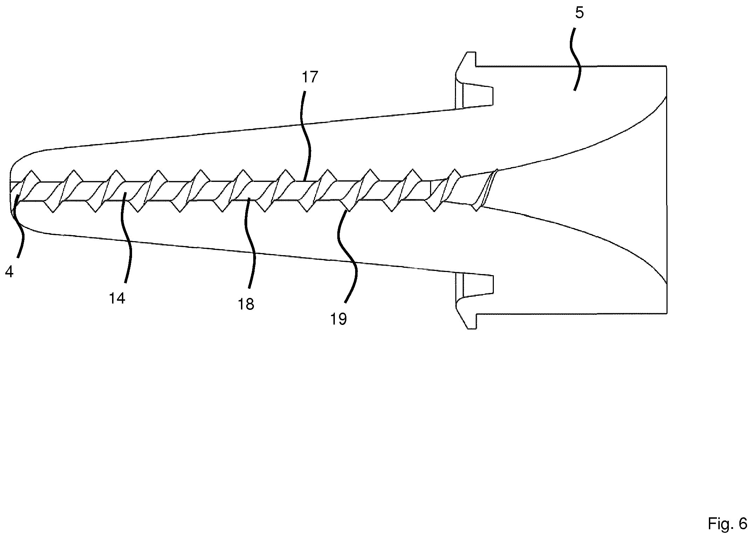

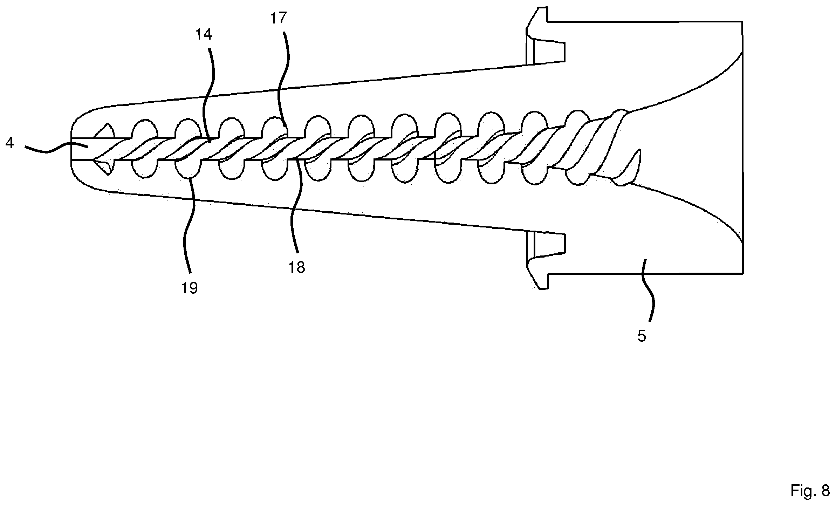

Die Fig. 6 bis 9 zeigen Ausführungsformen der Nadeldüse 5 mit unterschiedlich ausgestaltetem Nadelkanal 14.

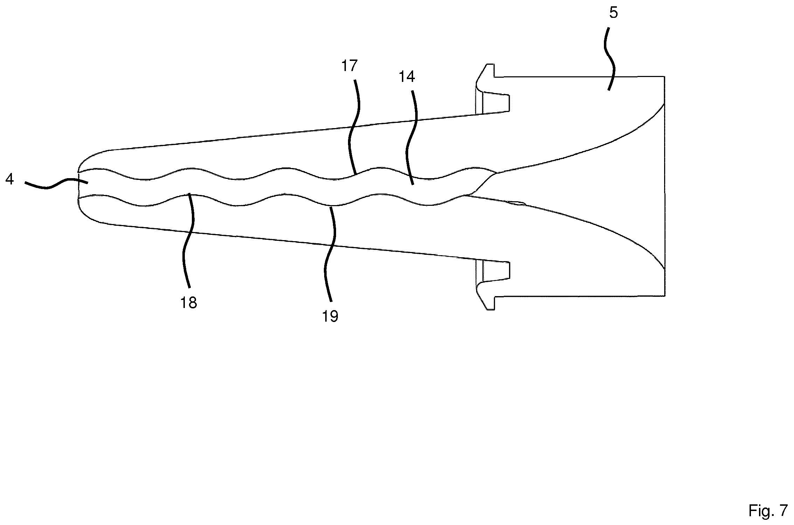

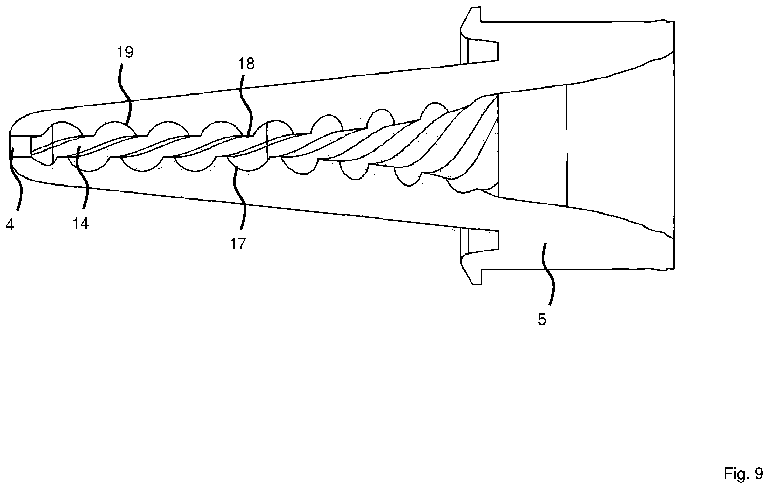

Bei den Ausführungsformen in den Fig. 4 bis 9 sind die proximalen Führungswandabschnitte 18 und / oder die distalen Wandabschnitte 19 entlang umlaufender Spirallinien ausgebildet, die als Schraubenlinie ausgeführt sein können. Fig. 7 zeigt eine Ausführungsform bei der die Kanalwand 17 des Nadelkanals 14 mit einer Wellenform gebildet ist, beispielsweise einer Sinuswellenform. Bei der Ausführung in Fig. 9 ändert sich die Steigung entlang des Nadelkanals 14, d.h. dort ist eine Spirallinie mit sich ändernder Steigung vorgesehen.

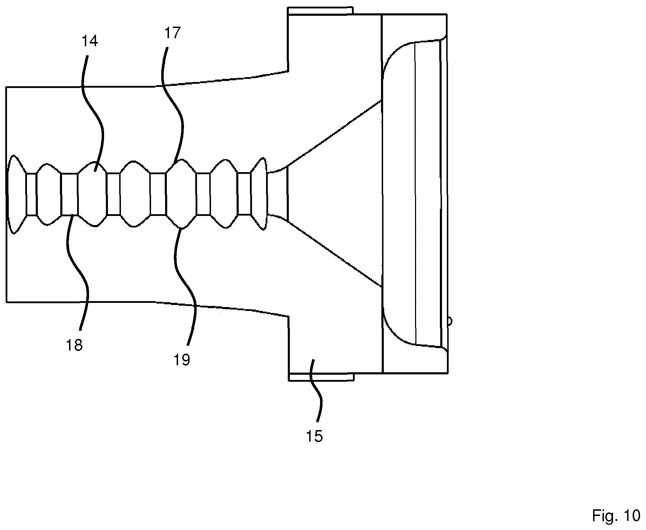

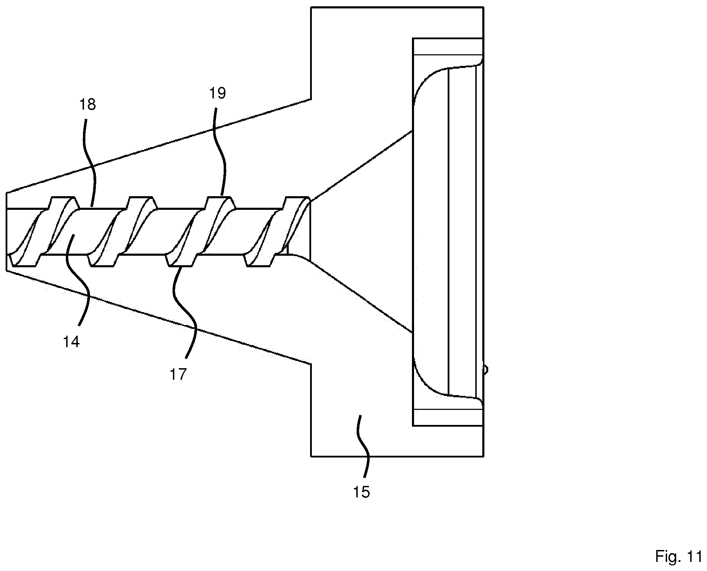

Die Fig. 10 und 11 zeigen Ausführungsformen des Nadelführungsbauteils 15, welches zur Ausbildung des Nadelkanals 14 in dem Nadelmodul 6 angeordnet ist, beispielsweise den Ausführungsformen in den Fig. 2 oder 3 entsprechend.

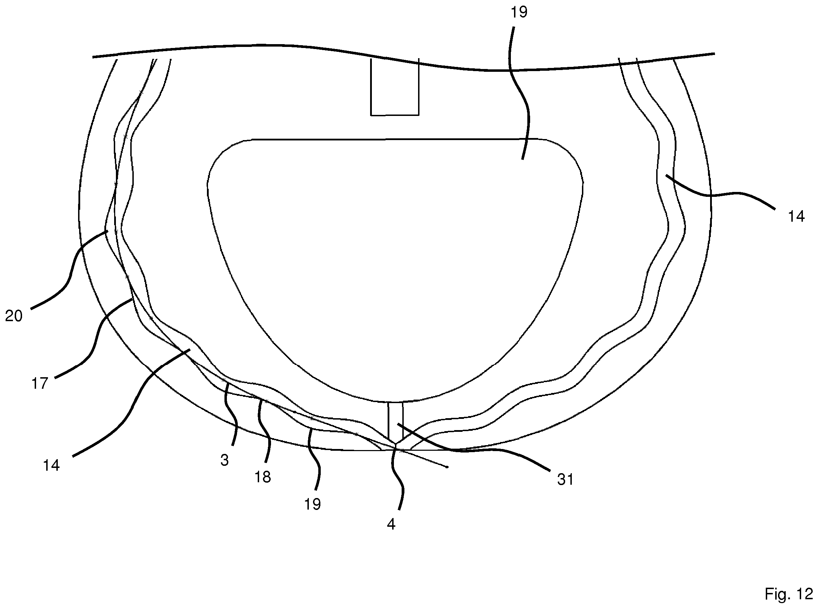

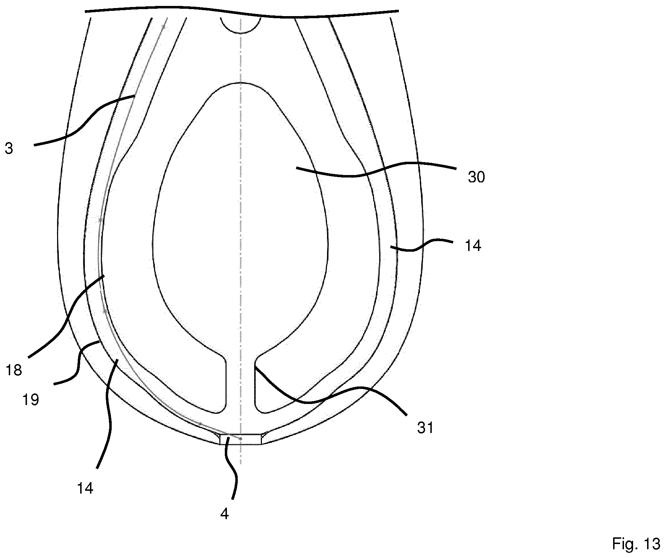

Die Fig. 12 und 13 zeigen einen vorderen Abschnitt eines Nadelmoduls 6, bei dem auf beiden Seiten eines Fluidreservoirs 30 der Nadelkanal 14 ausgebildet ist. In dem Nadelkanal 14 ist die Hautstechnadel 3 angeordnet, die zum Beispiel im Querschnitt ein Fachprofil aufweist. Die Nadelöffnung 4 steht über einen Fluidkanal 31 in Fluidverbindung mit dem Fluidreservoir 30.

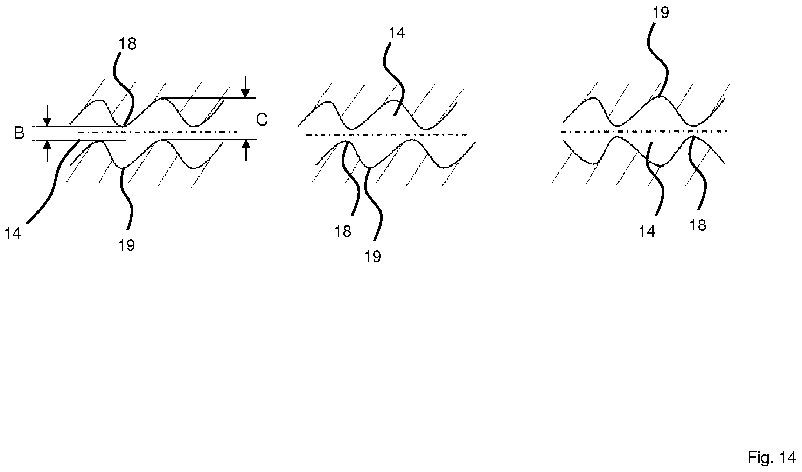

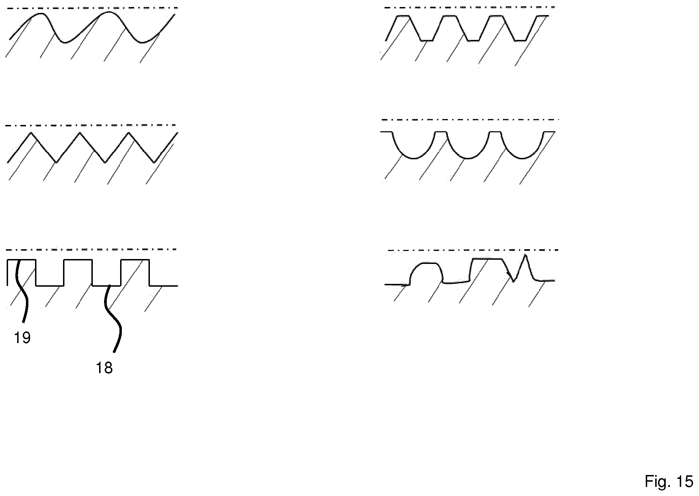

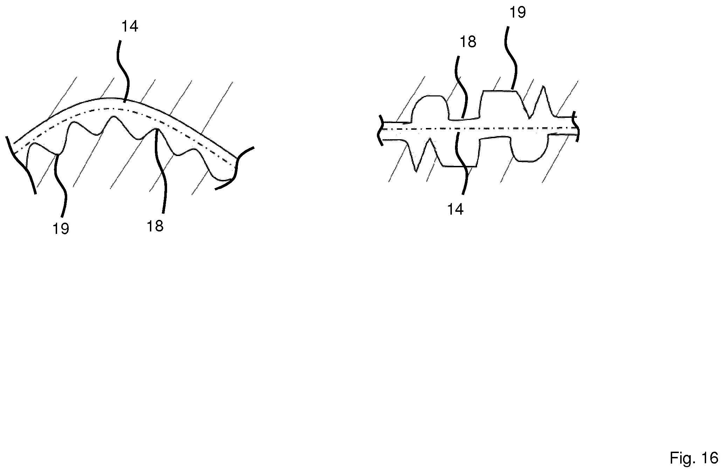

In den Fig. 14 bis 16 sind weitere Ausführungsformen für den Nadelkanal 14 gezeigt. Eine effektive Kanalbreite ist bestimmt durch den Abstand quer zur Längsrichtung des Nadelkanals 14 zwischen den auf gegenüberliegenden Seiten angeordneten proximalen Führungswandabschnitten 18. Unterschiedliche Querschnittsformen können vorgesehen sein, beispielsweise rund oder eckig.

Eine effektive Kanalbreite B (in der Schnittdarstellung) des Nadelkanals 14, die durch einen Abstand quer zur Längsrichtung des Nadelkanals 14 von proximalen Führungswandabschnitten 18 auf gegenüberliegenden Seiten des Nadelkanals 14 bestimmt ist (vgl. Fig. 14), kann größer als eine Dicke der Hautstechnadel 3 sein, insbesondere derart, dass ein leichtgängiges Bewegen der Hautstechnadel 3 in dem Nadelkanal 14 gegeben ist, insbesondere auch dann, wenn entlang des Nadelkanals 14 das Fluid transportiert wird. Die effektive Kanalbreite kann über die Länge des Nadelkanals 14 gleichbleibend sein. Alternativ kann sich die effektive Kanalbreite auf der Länge des Nadelkanals 14 ändern.

In Fig. 14 ist weiterhin (in der Schnittdarstellung) eine Breite C eines ausgetragenen Volumens des Fluids schematisch gezeigt.

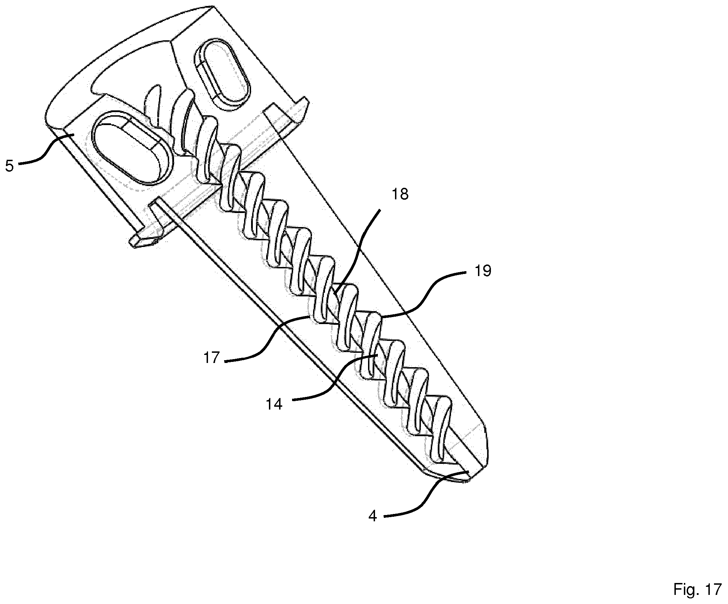

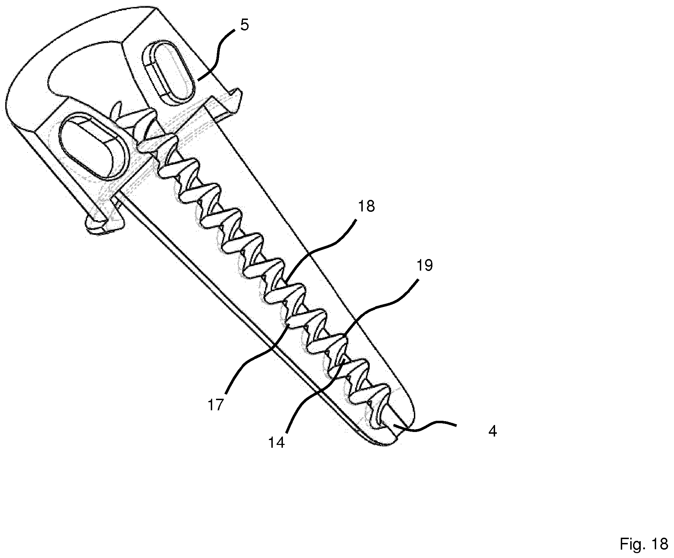

Die Fig. 17 und 18 zeigen jeweils eine schematische perspektivische Darstellung von Ausführungen der Nadeldüse 5 mit Nadelkanal 14, wobei die proximalen Führungswandabschnitte 18 und / oder die distalen Wandabschnitte 19 entlang einer Spirallinie (Fig. 17) oder entlang einer mäanderartigen Linie (Fig. 18) angeordnet sind.

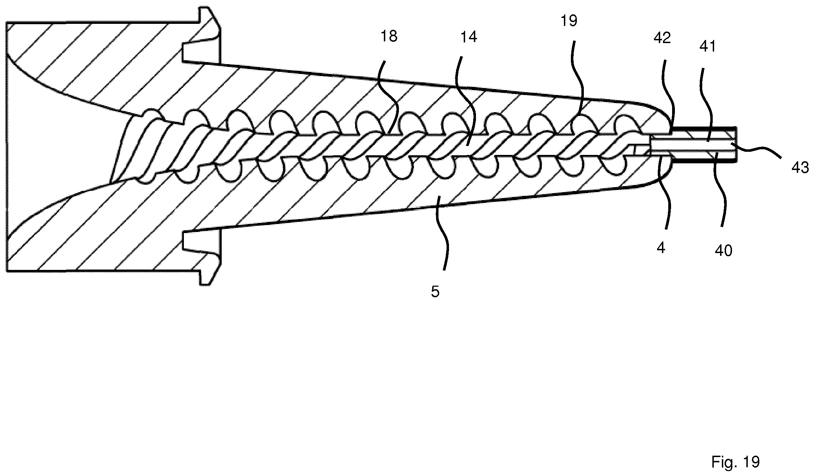

Fig. 19 zeigt eine alternative Ausführungsform der Nadeldüse 5, bei der im Bereich der Nadelöffnung 4 ein Nadelführelement 40 angeordnet ist, welches in der gezeigten Ausführung beispielhaft als Vorsatz oder Aufsatz ausgeführt ist und in welchem die Hautstechnadel 3 (nicht dargestellt) beim Vor- und Zurückbewegen in einer Verlängerung 41 des Nadelkanals 14 geführt ist. Das Nadelführungselement 40 steht von der Nadeldüse 5 ab und ist bei der gezeigten Ausführungsform mit einem proximalen Ende 42 in die Nadelöffnung 4 eingesetzt oder eingesteckt. Alternative kann das proximale Ende 42 auf das Nadelführelement 40 aufgesetzt oder einstückig an der Nadeldüse 5 angeformt sein. Eine Austrittsöffnung 43 für die Hautstechnadel 3 ist so versetzt zur Nadelöffnung 4 angeordnet. Bei der gezeigten Ausführung ist das Nadelführelement 40 frei von proximalen Führungswandabschnitte 18 und distalen Wandabschnitte 19 gebildet.

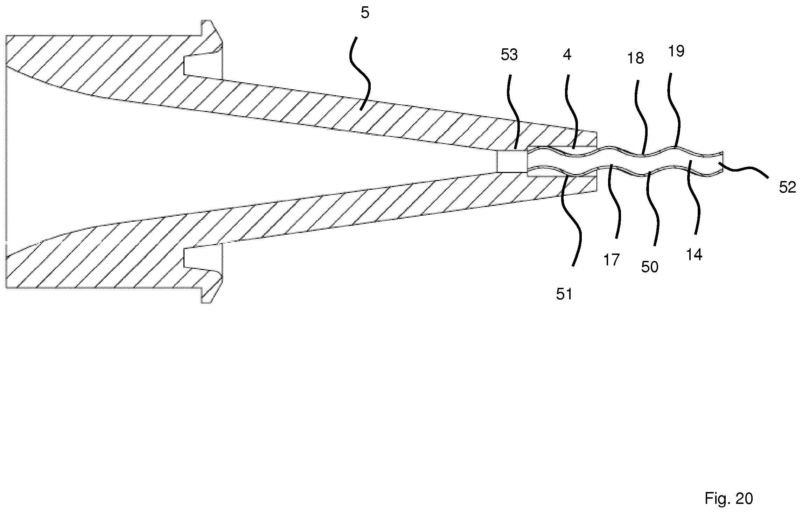

Fig. 20 zeigt eine weitere alternative Ausführungsform der Nadeldüse 5, bei der im Bereich der Nadelöffnung 4 ein Nadelführelement 50 angeordnet ist, welches als Ansatz, Einsatz oder Aufsatz ausgeführt sein kann und in welchem die Hautstechnadel 3 beim Vor- und Zurückbewegen in dem Nadelkanal 14 geführt ist. Das Nadelführelement 50 steht von der Nadeldüse 5 nach vorn ab oder über und ist bei der gezeigten Ausführungsform mit einem proximalen Ende 51 in die Nadelöffnung 4 eingesetzt. Alternativ kann das Nadelführelement 50 aufgesetzt sein. Auch kann eine einstückige Ausführung mit der Nadeldüse 5 vorgesehen sein. An dem Nadelführelement 50 sind im Bereich der Kanalwand 17 die proximalen Führungswandabschnitte 18 und die distalen Wandabschnitte 19 entlang des Nadelkanals 14 gebildet. Eine Austrittsöffnung 52 für die Hautstechnadel 3 ist versetzt (vorgelagert) zur Nadelöffnung 4 angeordnet. Im Abschnitt mit der Nadelöffnung 4 ist rückseitig ein Führungsabschnitt 53 angeordnet, welcher an das Nadelführungselement 50 anschließt. Alternativ kann ein Abstand hierzwischen vorgesehen sein.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Nadelführungsvorrichtung für eine Hautstecheinrichtung (1), mit
- einem Basiskörper;
- einer Hautstechnadel (3) und
- einem Nadelkanal (14), welcher in dem Basiskörper gebildet und in dem die Hautstechnadel (3) angeordnet ist, wobei die Hautstechnadel (3) in dem Nadelkanal (14) entlang einer Bewegungsbahn repetierend hin und her bewegbar ist;
wobei eine Kanalwand (17) des Nadelkanals (14) entlang der Bewegungsbahn in Bezug auf die Hautstechnadel (3) proximale Führungswandabschnitte (18), an denen die Hautstechnadel (3) beim Hin- und Herbewegen zur Nadelführung zur Anlage kommt, und distale Wandabschnitte (19) aufweist, die gegenüber den proximalen Führungswandabschnitten (18) zurückversetzt und beim Hin- und Herbewegen der Hautstechnadel (3) im Nadelkanal (14) frei von einem Berührungskontakt mit der Hautstechnadel (3) sind,
**dadurch gekennzeichnet, dass** die proximalen Führungswandabschnitte (18) und die distalen Wandabschnitte (19) in dem Nadelkanal (14) entlang der Bewegungsbahn abwechselnd gebildet und jeweils entlang einer oder mehrerer jeweils um den Nadelkanal (14) verlaufender Spirallinien sich vollständig um die Hautstechnadel herum erstreckend angeordnet sind, derart, dass entlang der distalen Wandabschnitte (19) eine durchgehende Fluidverbindung zwischen einem Kanaleintritt des Nadelkanals (14) und einem Kanalaustritt des Nadelkanals (14) ausgebildet ist.

2. Nadelführungsvorrichtung nach mindestens einem der vorangehenden Ansprüche, **dadurch gekennzeichnet, dass** der Nadelkanal (14) als ein Fluidtransportkanal ausgeführt ist.

3. Nadelführungsvorrichtung nach Anspruch 2, **dadurch gekennzeichnet, dass** den distalen Wandabschnitten (19) vorgelagerte Volumina (20) entlang der Bewegungsbahn in Fluidverbindung stehen.

4. Nadelführungsvorrichtung nach mindestens einem der vorangehenden Ansprüche, **dadurch gekennzeichnet, dass** die Bewegungsbahn einen geraden Abschnitt in einem geraden Nadelkanalabschnitt und / oder einen gekrümmten Abschnitt in einem gekrümmten Nadelkanalabschnitt aufweist.

5. Nadelführungsvorrichtung nach mindestens einem der vorangehenden Ansprüche, **dadurch gekennzeichnet, dass** die proximalen Führungswandabschnitte (18) eine flächige und / oder eine punktförmige Kontaktfläche aufweisen, derart, dass beim Führen der Hautstechnadel (3) in dem Nadelkanal (14) diese mit dem proximalen Führungswandabschnitt (18) einen flächigen und / oder einen punktförmigen Berührungskontakt ausbildet.

6. Nadelführungsvorrichtung nach mindestens einem der vorangehenden Ansprüche, **dadurch gekennzeichnet, dass** die proximalen Führungswandabschnitte (18) und / oder die distalen Wandabschnitte (19) in einander gegenüberliegenden Kanalwandabschnitten entlang der Bewegungsbahn versetzt zueinander ausgebildet sind.

7. Nadelführungsvorrichtung nach mindestens einem der vorangehenden Ansprüche, **dadurch gekennzeichnet, dass** der Nadelkanal (14) mit einem Fluidreservoir (30) verbunden ist.

8. Nadelführungsvorrichtung nach mindestens einem der vorangehenden Ansprüche, **dadurch gekennzeichnet, dass** eine effektive Kanalbreite des Nadelkanals (14), die durch einen Abstand quer zur Längsrichtung des Nadelkanals (14) von proximalen Führungswandabschnitten (18) auf gegenüberliegenden Seiten des Nadelkanals bestimmt ist, größer als eine Dicke der Hautstechnadel (3) ist.

9. Nadelführungsvorrichtung nach mindestens einem der vorangehenden Ansprüche, **dadurch gekennzeichnet, dass** der Nadelkanal (14) zumindest teilweise in einer Nadeldüse (5) ausgebildet ist.

10. Nadelführungsvorrichtung nach mindestens einem der vorangehenden Ansprüche, **dadurch gekennzeichnet, dass** der Nadelkanal (14) zumindest teilweise in einem Nadelführungsbauteil (15) eines Nadelmoduls (6) für eine Haustecheinrichtung (1) ausgebildet ist.

11. Nadelführungsvorrichtung nach mindestens einem der vorangehenden Ansprüche, **dadurch gekennzeichnet, dass** der Basiskörper mit dem Nadelkanal (14) als ein Einwegbauteil ausgeführt ist.

12. Hautstecheinrichtung, mit einem Antriebsmodul (7) und einem hieran gekoppelten Nadelmodul (6), wobei das Nadelmodul (6) eine Nadelführungsvorrichtung nach mindestens einem der vorangehenden Ansprüche aufweist.

## Claims

1. Needle guide device for a skin piercing device (1), comprising
- a base body;
- a skin piercing needle (3) and
- a needle channel (14) which is formed in the base body and in which the skin piercing needle (3) is arranged, wherein the skin piercing needle (3) can be moved back and forth in a repeating manner in the needle channel (14) along a movement path;
wherein a channel wall (17) of the needle channel (14) has, along the movement path in relation to the skin piercing needle (3), proximal guide wall portions (18), against which the skin piercing needle (3) comes to bear during the movement back and forth for needle guidance, and distal wall portions (19) which are set back in relation to the proximal guide wall portions (18) and are free from a physical contact with the skin piercing needle (3) during the movement back and forth of the skin piercing needle (3) in the needle channel (14),
**characterized in that** the proximal guide wall portions (18) and the distal wall portions (19) are formed alternately in the needle channel (14) along the movement path and are respectively arranged extending completely around the skin piercing needle along one or more spiral lines respectively running around the needle channel (14) in such a way that a continuous fluid connection is formed along the distal wall portions (19) between a channel inlet of the needle channel (14) and a channel outlet of the needle channel (14).

2. Needle guiding device according to at least one of the preceding claims, **characterized in that** the needle channel (14) is configured as a fluid transport channel.

3. Needle guiding device according to Claim 2, **characterized in that** volumes (20) in front of the distal wall portions (19) are in fluid connection along the movement path.

4. Needle guiding device according to at least one of the preceding claims, **characterized in that** the movement path has a straight portion in a straight needle channel portion and/or a curved portion in a curved needle channel portion.

5. Needle guiding device according to at least one of the preceding claims, **characterized in that** the proximal guide wall portions (18) have a planar and/or punctiform contact surface in such a way that, when the skin piercing needle (3) is guided in the needle channel (14), said needle forms a planar and/or punctiform contact with the proximal guide wall portion (18).

6. Needle guiding device according to at least one of the preceding claims, **characterized in that** the proximal guide wall portions (18) and/or the distal wall portions (19) are formed offset with respect to one another in mutually opposite channel wall portions along the movement path.

7. Needle guiding device according to at least one of the preceding claims, **characterized in that** the needle channel (14) is connected to a fluid reservoir (30).

8. Needle guiding device according to at least one of the preceding claims, **characterized in that** an effective channel width of the needle channel (14), which is determined by a distance transverse to the longitudinal direction of the needle channel (14) from proximal guide wall portions (18) on opposite sides of the needle channel, is greater than a thickness of the skin piercing needle (3).

9. Needle guiding device according to at least one of the preceding claims, **characterized in that** the needle channel (14) is formed at least partially in a needle nozzle (5).

10. Needle guiding device according to at least one of the preceding claims, **characterized in that** the needle channel (14) is formed at least partially in a needle guiding component (15) of a needle module (6) for a skin piercing device (1).

11. Needle guiding device according to at least one of the preceding claims, **characterized in that** the base body with the needle channel (14) is configured as a disposable component.

12. Skin piercing device, comprising a drive module (7) and a needle module (6) coupled thereto, wherein the needle module (6) comprises a needle guiding device according to at least one of the preceding claims.

## Revendications

1. Dispositif de guidage d'aiguille pour dispositif de perforation cutanée (1), avec
- un corps de base ;
- une aiguille de piquage de la peau (3) et
- un canal d'aiguille (14) formé dans le corps de base et dans lequel est placée l'aiguille de piquage de la peau (3), l'aiguille de piquage de la peau (3) pouvant se déplacer en va-et-vient de manière répétitive dans le canal d'aiguille (14) le long d'une trajectoire de mouvement ;
dans lequel une paroi de canal (17) du canal d'aiguille (14) présentant, le long de la trajectoire de mouvement par rapport à l'aiguille de piquage de la peau (3), des sections de paroi de guidage proximales (18) avec lesquelles l'aiguille de piquage de la peau (3) vient en contact lors du déplacement en va-et-vient pour le guidage de l'aiguille, et des sections de paroi distales (19) qui sont décalées par rapport aux sections de paroi de guidage proximales (18) et qui, lors du déplacement en va-et-vient de l'aiguille de piquage de la peau (3) dans le canal d'aiguille (14), sont exemptes de contact avec l'aiguille de piquage de la peau (3),
**caractérisé en ce que** les sections de paroi de guidage proximales (18) et les sections de paroi distales (19) sont alternativement formées dans le canal d'aiguille (14) le long de la trajectoire de mouvement et sont disposées respectivement autour du canal d'aiguille (14) le long d'une ou plusieurs lignes spiralées de manière à s'étendre entièrement autour de l'aiguille de piquage de la peau, de telle sorte qu'une liaison fluidique continue soit établie le long des sections de paroi distales (19) entre une entrée de canal du canal d'aiguille (14) et une sortie de canal du canal d'aiguille (14).

2. Dispositif de guidage d'aiguille selon au moins une des revendications précédentes, **caractérisé en ce que** le canal d'aiguille (14) est réalisé sous la forme d'un canal de transport de fluide.

3. Dispositif de guidage d'aiguille selon la revendication 2, **caractérisé en ce que** des volumes (20) situés en amont des sections de paroi distales (19) sont en connexion fluidique le long de la trajectoire de mouvement.

4. Dispositif de guidage d'aiguille selon au moins une des revendications précédentes, **caractérisé en ce que** la trajectoire de mouvement comporte une section droite dans une section de canal d'aiguille droite et / ou une section incurvée dans une section de canal d'aiguille incurvée.

5. Dispositif de guidage d'aiguille selon au moins l'une des revendications précédentes, **caractérisé en ce que** les sections de paroi de guidage proximales (18) ont une surface de contact superficielle et / ou ponctuelle, de telle sorte que, lors du guidage de l'aiguille de piquage de la peau (3) dans le canal d'aiguille (14), celle-ci établie un contact superficiel et / ou ponctuel avec la section de la paroi de guidage proximale (18).

6. Dispositif de guidage d'aiguille selon au moins une des revendications précédentes, **caractérisé en ce que** les sections de paroi de guidage proximales (18) et / ou les sections de paroi distales (19) sont formées dans des sections de paroi de canal opposées le long de la trajectoire de mouvement de manière à être décalées les unes par rapport aux autres.

7. Dispositif de guidage d'aiguille selon au moins une des revendications précédentes, **caractérisé en ce que** le canal d'aiguille (14) est relié à un réservoir de fluide (30).

8. Dispositif de guidage d'aiguille selon au moins une des revendications précédentes, **caractérisé en ce qu'**une largeur effective du canal d'aiguille (14), qui est déterminée par une distance transversale à la direction longitudinale du canal d'aiguille (14) des sections de paroi de guidage proximales (18) sur les côtés opposés du canal d'aiguille, est supérieure à une épaisseur de l'aiguille de piquage de la peau (3).

9. Dispositif de guidage d'aiguille selon au moins une des revendications précédentes, **caractérisé en ce que** le canal d'aiguille (14) est formé au moins partiellement dans une buse à aiguille (5).

10. Dispositif de guidage d'aiguille selon au moins une des revendications précédentes, **caractérisé en ce que** le canal d'aiguille (14) est au moins partiellement formé dans un composant de guidage d'aiguille (15) d'un module d'aiguille (6) pour un dispositif de piquage de la peau (1).

11. Dispositif de guidage d'aiguille selon au moins une des revendications précédentes, **caractérisé en ce que** le corps de base est conçu avec le canal d'aiguille (14) en tant que composant à usage unique.

12. Dispositif de piquage de peau, comprenant un module d'entraînement (7) et un module d'aiguille (6) couplé à celui-ci, le module d'aiguille (6) présentant un dispositif de guidage d'aiguilles selon au moins l'une des revendications précédentes.
